# EUROPÄISCHE PATENTSCHRIFT

(11) **EP 0 530 455 B1**
(45) Veröffentlichungstag und Bekanntmachung des Hinweises auf die Patenterteilung: **27.12.1995**
(21) Anmeldenummer: 92110495.6
(22) Anmeldetag: 22.06.1992
(51) Int. Cl.: A61K 35/10

(54) **Huminate enthaltende Nasenarznei**
Huminate containing nasal agent
Médicament nasal contenant un huminate

(30) Priorität: 04.09.1991 DE 4129396
(43) Veröffentlichungstag der Anmeldung: 10.03.1993
(73) Patentinhaber: RÜTGERSWERKE AKTIENGESELLSCHAFT, 60326 Frankfurt (DE)
(72) Erfinder: Seubert, Bernhard, W-6805 Edingen-Neckarhausen 1 (DE); Gubbe, Ute, W-6834 Ketsch (DE); Haase, Anton, Dr., W-6109 Mühltal 1 (DE)

(56) Entgegenhaltungen:
- EP-A- 0 281 678
- EP-A- 0 281 679
- EP-A- 0 283 573
- EP-A- 0 313 718

## Beschreibung

Die Erfindung betrifft die Verwendung niedermolekularer Ammonium- oder Alkalihuminate zur Herstellung einer Nasenarznei, insbesondere Nasenspray oder -tropfen gegen Heuschnupfen.

Heuschnupfen ist eine weitverbreitete allergische Reaktion des menschlichen Körpers, insbesondere der Schleimhäute im Nasen- und Augenbereich auf die Pollen bestimmter Pflanzen, besonders auf Pollen von Gräsern und Getreidearten. Die Allergie beeinträchtigt das Wohlbefinden und die Leistungsfähigkeit der Betroffenen sehr stark. Man versucht daher, mit verschiedenen Methoden, dieser Krankheit Herr zu werden, oder zumindest ihre Symptome so zu unterdrücken, daß auch der Aufenthalt der Patienten in der freien Natur ohne Beschwerden möglich ist.

Viele Patienten wollen sich dazu aber weder einer langwierigen Desensibilisierungsbehandlung unterziehen, noch Medikamente einnehmen, die unter Umständen unerwünschte Nebenwirkungen haben und deren erwünschte Wirkung erst nach relativ langer Zeit nach der Wirkstoffeinnahme einsetzt. Gewünscht ist vielmehr eine Arznei, die beim Auftreten der Symptome appliziert wird und die sofort Linderung verschafft. Dies ist durch entsprechende Nasenarzneien, -sprays oder -tropfen möglich.

Bekannt sind Nasenarzneien, die gegen Heuschnupfen empfohlen werden, auf Basis antiallergisch wirkender Substanzen wie z. B. Chromoglicinsäure oder Hydroxyphenylalkanolaminen sowie solche auf Basis schleimhautabschwellender Substanzen wie z. B. Cortisonpräparaten oder von Imidazol-Derivaten. Erstere haben den Nachteil, daß sie bei vielen Patienten nicht oder nur sehr schwach wirksam sind und die zweite Gruppe läßt bei längerzeitlicher Anwendung Nebenwirkungen erwarten.

Es ist daher Aufgabe der Erfindung, ein gut verträgliches Mittel bereitzustellen, das als Nasenspray oder -tropfen angewandt werden kann und das bei einem großen Personenkreis wirksam und rasch die Symptome des Heuschnupfens reduziert und auch bei längerer Anwendung keine unerwünschte oder schädliche Nebenwirkung zeigt.

Die Lösung der Aufgabe erfolgt durch eine Nasenarznei gemäß dem Anspruch 1.

Es wurde gefunden, daß eine verdünnte, wäßrige Lösung niedermolekularer Ammonium- oder Alkalihuminate, wenn sie in den Nasenbereich eines unter Heuschnupfen leidenden Patienten eingebracht wird, die Symptome schnell und wirksam lindert, ohne daß irgendwelche negativen Nebeneffekte auftreten. Die Schwellung und Reizung im Nasenbereich wird reduziert und auch die Symptome im Augenbereich werden stark gelindert. Überraschenderweise tritt nach mehrtägiger Anwendung einer entsprechenden Huminatlösung eine zusätzliche Verbesserung dahingehend auf, daß sich die Wirkungszeit nach einer Applikation verlängert, bzw. daß an Tagen mit geringerem Pollenflug keine Anwendung der Huminatlösung notwendig ist. Dabei kann diese wäßrige Lösung, die 0,1 bis 3 Gew. % niedermolekularer Huminate und ggf. noch weitere, medizinisch wirksame Substanzen sowie Konservierungsmittel enthält, sowohl als Nasenspray verwendet werden, wie auch als Nasentropfen oder sogar als Nasengel. Bei Einsatz als Nasentropfen ist es zweckmäßig, bei Einsatz als Nasengel unabdinglich, daß die Lösung mit einem üblichen Verdickungsmittel wie etwa Bentonit, hochdisperser Kieselsäure, Stärke oder Gelatine bis zur gewünschten Konsistenz verdickt ist. In den erfindungsgemäßen Nasenarzneien einsetzbar und wirksam sind sowohl natürliche niedermolekulare Ammonium- oder Alkalihuminate, wie sie aus DE-A 37 07 909 oder DE-A 37 36 623 bekannt sind, als auch synthetisch durch Oxidation von mehrwertigen Phenolen in schwach alkalischem wäßrigen Medium entsprechend DE-A 37 07 910 hergestellte niedermolekulare Ammonium- oder Alkalihuminate. Es sind dies dunkelbraune, wasserlösliche Produkte mit einem mittleren Molekulargewicht von 1.000, bei einem Streubereich von 300 bis 1.500.

Von diesen Wirkstoffen ist bekannt, daß sie als Heilmittel in der Wundheilung wirksam sind. Trotzdem ist es überraschend, daß sich diese Stoffe in geringer Konzentration zur Linderung der Symptome des Heuschnupfens eignen.

### Beispiel

Eine Versuchsgruppe von 10 Personen, die unter durch Pollenflug bedingten allergischen Reaktionen insbesondere im Nasen- und Augenbereich leiden, verwendeten eine 0,5 %ige wäßrige Lösung eines Natriumhuminates mit einem mittleren Molekulargewicht von 1.000, bei einem Streuberich von 300 bis 1.500 als Nasenspray, indem sie sich bei Auftreten der Heuschnupfensymptome mittels einer an sich bekannten und üblicherweise für Nasensprays verwendeten Vorratsflasche jeweils eine Dosis der Lösung in die Nase sprühten. Bei allen Patienten verringerten sich innerhalb von Minuten die Beschwerden. Die Nasen wurden frei, der Niesreiz verschwand, die Verquellung der Augen wurde reduziert. Unerwünschte Nebenwirkungen wurden auch nach längerem Gebrauch des Mittels nicht beobachtet. Hingegen berichteten sechs der Versuchspersonen, daß nach mehrtägigem Gebrauch des Mittels die Zahl der notwendigen Anwendungen sich wesentlich reduziert hat. An Tagen mit niedriger Pollenbelastung waren diese Patienten auch ohne das erfindungsgemäße Nasenspray beschwerdefrei.

## Patentansprüche

1. Verwendung niedermolekularer Ammonium- oder Alkalihuminate mit einem mittleren Molekulargewicht von 1.000 bei einem Streubereich von 300 bis 1.500 zur Herstellung einer Nasenarznei gegen Heuschnupfen.

## Claims

1. Use of low-molecular ammonium or alkali huminates with an average molecular weight of 1000 with a scatter of from 300 to 1500 to produce a nasal medicament to combat hay fever.

## Revendications

1. Utilisation d'un huminate d'ammoinium ou alcalin avec un faible poids moléculaire moyen de 1000 pour un domaine de dispersion de 300 à 1500 pour la préparation d'un médicament nasal contre le rhume des foins.
